(19) Europäisches Patentamt
European Patent Office
Office européen des brevets

(11) EP 4 295 698 A1

(12) **EUROPEAN PATENT APPLICATION**
published in accordance with Art. 153(4) EPC

(43) Date of publication:
**27.12.2023 Bulletin 2023/52**

(21) Application number: **22734769.7**

(22) Date of filing: **28.02.2022**

(51) International Patent Classification (IPC):
***A23K 20/20*** *(2016.01)*

(86) International application number:
**PCT/CN2022/078264**

(87) International publication number:
**WO 2022/144044 (07.07.2022 Gazette 2022/27)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.04.2021 CN 202110372778**

(71) Applicant: **Guangzhou Insighter Biotechnology Co., Ltd**
**Guangzhou, Guangdong 510663 (CN)**

(72) Inventor: **PENG, Xianfeng**
**Guangzhou, Guangdong 510663 (CN)**

(74) Representative: **Murgitroyd & Company**
**Murgitroyd House**
**165-169 Scotland Street**
**Glasgow G5 8PL (GB)**

(54) **ASPARTIC ACID COPPER COMPLEX, AND USE THEREOF**

(57) An aspartic acid copper complex, a use thereof, and a feed composition containing said aspartic acid copper complex. The chemical structure of the aspartic acid copper complex is $[(Cu(II))(Asp)(H_2O)_m]\cdot(H_2O)_n$, wherein Asp is L-Asp or DL-Asp, m is any integer from 0 to 10, and n is any value from 0 to 10. The present aspartic acid copper complex is applied to animal breeding. Calculated in terms of the copper element, a physiological requirement usage amount may promote the growth of livestock and poultry at all stages of the growth cycle, and during high dose use, and the growth performance of the animals is normal under high-dose use, thereby overcoming problems such as excessive use of high-dose inorganic copper in the breeding industry being harmful for animals.

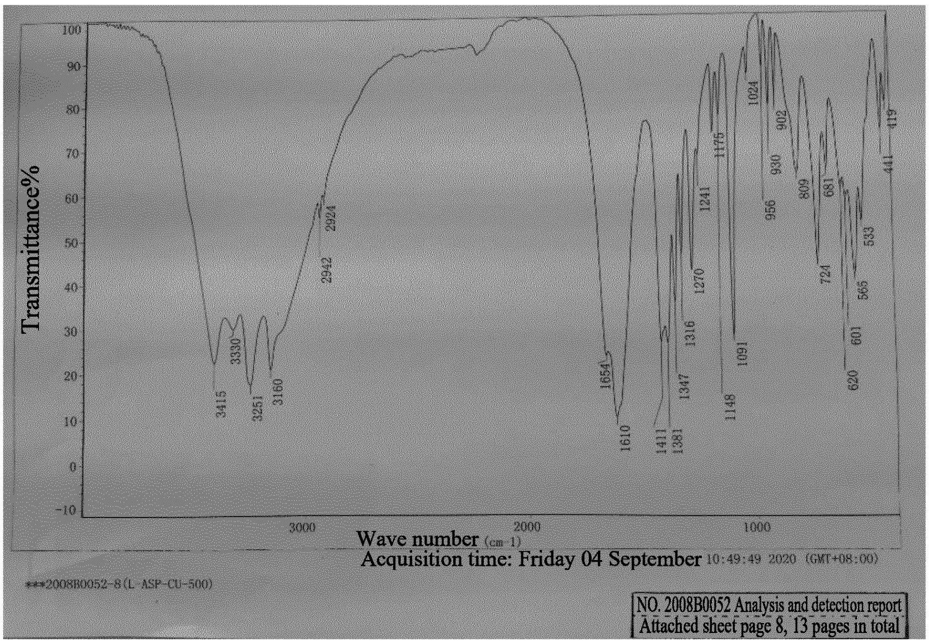


*FIG. 1*



EP 4 295 698 A1

## Description

### Technical Field

The present application relates to the field of animal feed additives, and specifically to an aspartic acid copper complex and the use thereof in the preparation of an animal feed additive.

### Background Art

Copper is one of the essential trace elements for animals. It is a key and active centre of some enzymes, acts as a cofactor of various oxidases, and is a component of coagulation factor V and MT in animals. Copper maintains the normal haematopoietic function, the normal structure of bone, blood vessels and skin, and the health of the central nervous system, protects the normal pigment and structure of the hair, and protects the cells of an organism from the toxicity of superoxide ions. In 1984, K. Kaemmerer reported that copper deficiency could significantly delay the growth of animals, and resupply of copper could quickly restore the growth of animals. The nutritional requirement for copper in animals ranges from 5 mg/kg to 8 mg/kg.

Since studies found in 1945 that the high levels of copper could significantly improve the growth performance of pigs, feeds with 200 mg/kg to 250 mg/kg of high-level copper had been used in the breeding of young piglets to improve their growth performance and shorten the breeding cycle. The availability of inorganic copper is related to its solubility, with higher solubility indicating greater availability. Among the various forms of inorganic copper, copper sulphate is the most preferred due to its comparable effects to organic copper and chelated copper. Therefore, in view of the cost advantage, inorganic copper, especially copper sulphate, has become the main copper source of high-level copper in piglet feeds. However, the high levels of copper have effects similar to antibiotics, significantly promoting growth in piglets bred under conventional conditions, but the high-level copper tends to hinder growth rather than promote it in pigs bred under sterile conditions. The solubility of the inorganic copper source itself or its ability to be rapidly decomposed by gastric acid in the pig's stomach leads to a swift release and absorption of a large amount of free copper ions in the stomach. This exceeds the physiological limit of the animal and leads to toxic reactions. A large amount of copper ions released in gastric acid can be absorbed due to the presence of phytic acid or fibre in the diet, resulting in only a small amount of copper ions reaching the middle and distal parts of the small intestine to perform an antimicrobial effect or be absorbed by the small intestine into an organism to exert their biological effects. Therefore, it is necessary to add a high concentration of copper ions in the feeds to achieve the antimicrobial and growth-promoting effects. Copper level exceeding the physiological limit can lead to numerous side effects, such as organ damage by high-level copper, food safety issues due to excessive residues in organs, interference with the absorption of other nutrients, and environmental pollution caused by unused copper excreted through faeces.

Sources of copper for animal consumption can be broadly divided into two categories: the first category includes inorganic copper, such as copper sulphate, copper chloride, copper oxide, copper acetate, copper carbonate and copper sulphide; the second category includes chelated copper or organic-inorganic copper complex, such as casein copper, milk protein copper, soybean protein copper, methionine copper, copper stearate, and copper lysine hydrochloride. The prior art also shows that as monogastric animals or poultry grow, inorganic copper or organic copper at high levels tends to have reduced effects on the production performance of the animals.

Aspartic acid is not essential for mammals and can be produced from oxaloacetic acid via transamination.

In view of the above, the present application is hereby filed.

### Summary of the Invention

The present application aims to provide an aspartic acid copper complex that is safe and can improve the production performance of an animal throughout the growth period.

The present application also aims to provide a feed composition comprising an aspartic acid copper complex that is safe and can improve the production performance of an animal throughout the growth period.

The present application also aims to provide the use of the aspartic acid copper complex and the feed composition thereof in the preparation of an animal feed additive.

The present application also aims to provide the use of the aspartic acid copper complex and the feed composition thereof in the preparation of an animal feed.

The present application further aims to provide a method for improving the production performance of an animal.

In order to achieve at least one of the objectives of the present application, the specific technical solutions are as follows.

In one aspect, the present application provides an aspartic acid copper complex having a chemical structure of $[(Cu(II))(Asp)(H_2O)_m]\cdot(H_2O)_n$, wherein Asp is L-Asp or DL-Asp, m is any integer from 0 to 10, and n is any value from

0 to 10.

In a technical solution, the aspartic acid copper complex has a chemical structure of $[(Cu(II))(Asp)(H_2O)_m]\cdot(H_2O)_n$, wherein Asp is L-Asp or DL-Asp, and n is any value from 0 to 0.62.

In some embodiments, the aspartic acid copper complex has any one of the chemical structures as shown below:

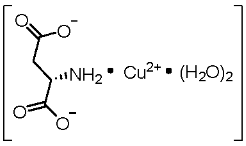,
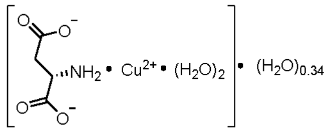,
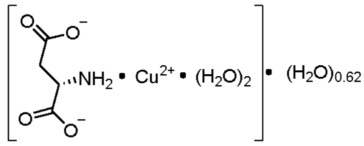,

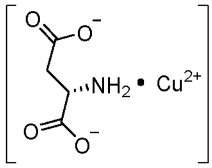,
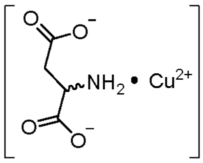,
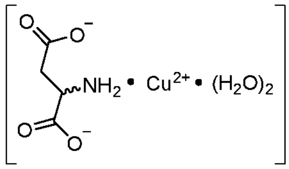,

and

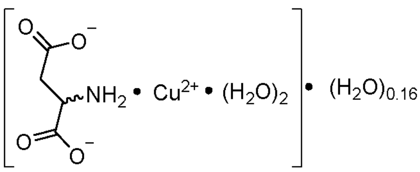.

In another aspect, the present application also provides a feed composition comprising at least one aspartic acid copper complex provided by the present invention, and at least one of the auxiliary agents acceptable in feeds, pharmacy or foods.

In some technical solutions, the feed composition further comprises an additional animal feed additive.

The additional animal feed additive comprises a nutritive feed additive, a non-nutritive feed additive and a medicinal feed additive.

In other technical solutions, the feed composition further comprises a feed raw material.

In another aspect, the present application also provides the use of an aspartic acid copper complex having a chemical structure of $[(Cu(II))(Asp)(H_2O)_m]\cdot(H_2O)_n$ and a feed composition thereof in the preparation of an animal feed additive.

In some technical solutions, the animal is livestock, poultry, aquatic animals or pets at all stages of growth.

In another aspect, the present application also provides the use of an aspartic acid copper complex having a chemical structure of $[(Cu(II))(Asp)(H_2O)_m]\cdot(H_2O)_n$ and a feed composition thereof in the preparation of an animal feed.

In some technical solutions, the animal is livestock, poultry, aquatic animals or pets at all stages of growth.

In another aspect, the present application further provides a method for improving the production performance of an animal, the method comprising: feeding the animal with a feed comprising the aspartic acid copper complex having a chemical structure of $[(Cu(II))(Asp)(H_2O)_m]\cdot(H_2O)_n$ provided by the present invention and the feed composition thereof; alternatively, adding an aspartic acid copper complex having a chemical structure of $[(Cu(II))(Asp)(H_2O)_m]\cdot(H_2O)_n$, or a feed composition or feed additive comprising the aspartic acid copper complex having a chemical structure of $[(Cu(II))(Asp)(H_2O)_m]\cdot(H_2O)_n$ to the diet of the animal in an amount required for animal growth and feeding the animal with the diet, wherein the usage amount of the aspartic acid copper complex, or the feed composition or animal feed additive, calculated in terms of copper element and based on the weight of the animal diet, is 5 mg/kg to 300 mg/kg.

Compared with the prior art, the present application has beneficial effects that include:

in the present invention, it is found that the aspartic acid copper complex having a chemical structure of $[(Cu(II))(Asp)(H_2O)_m]\cdot(H_2O)_n$, when applied to animal breeding and calculated in terms of copper element, can promote the growth of livestock and poultry at all stages of the animal's growth even when only physiological necessary quantities are used, and the growth performance of the animals is normal under high-dose use and overcomes problems such as excessive use of high-level inorganic copper in the breeding industry being harmful for animals.

Any embodiment according to any aspect of the present application can be combined with other embodiments,

unless they are contradictory to each other. In addition, in any embodiment according to any aspect of the present application, any technical feature can be applied to the technical feature in other embodiments, unless they are contradictory to each other.

## Brief Description of the Drawings

FIG. 1 shows the infrared diffraction spectrum of the aspartic acid copper complex having a chemical formula of $[Cu(L\text{-}Asp)(H_2O)_2]$.

FIG. 2 shows the infrared diffraction spectrum of the mixture of the preparation raw materials of the aspartic acid copper complex having a chemical formula of $[Cu(L\text{-}Asp)(H_2O)_2]$.

## Detailed Description of Embodiments

The foregoing content is merely a summary of certain aspects of the present application, but is not limited to these aspects. The content involved above and in other aspects will be described more specifically and completely below.

Certain embodiments of the present application will now be described in detail, examples of which are illustrated by the accompanying structural formulas and chemical formulas. The present application intends to cover all alternative, modified and equivalent technical solutions, which are all included within the scope of the present application as defined by the claims. Additionally, for clarity, some technical features of the present application are described in multiple independent embodiments, respectively, but they may also be provided in combination or in the form of any suitable subcombination in a single embodiment.

The present invention provides an aspartic acid copper complex having a chemical structure of $[(Cu(II))(Asp)(H_2O)_m]\cdot (H_2O)_n$, wherein Asp is L-Asp or DL-Asp, m is any integer from 0 to 10, and n is any value from 0 to 10.

The term "complex" involved in the present invention refers to a stable substance formed by a copper ion of a certain soluble copper salt, an aspartate ion (chemical structure being $^-OOC\text{-}CH_2\text{-}CH(NH_2)\text{-}COO^-$, and abbreviated as "Asp" in the present invention) and water molecules in a contact process. The formation of the substance is due to the external and internal conditions that cause them to bind in certain chemical molar equivalent and/or non-chemical equivalent through covalent bonds and/or non-covalent intermolecular forces.

The term "and/or" as used herein comprises any and all combinations of one or more relevant items listed.

Aspartic acid (an English name), also known as asparaginic acid, has a chemical name of aminosuccinic acid and a chemical structure of $HOOC\text{-}CH_2\text{-}CH(NH_2)\text{-}COOH$. Aspartic acid contains asymmetric carbon atoms (denoted as "$-^*CH(NH_2)-$") and is therefore optically active. Based on the different spatial arrangement positions, aspartic acid can be divided into L-form

( 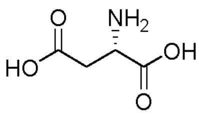 , formula I)

and D-form

( 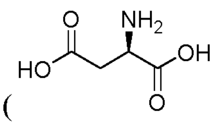 , formula II),

which are enantiomers of each other. D-aspartic acid and L-aspartic acid at equal amounts form an aspartic acid racemate that is not optically active, and the racemate is denoted as DL-form (

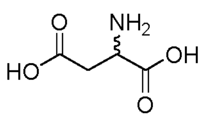 , formula III).

DL-aspartic acid can be an aspartic acid racemate mixture or a racemic aspartic acid. The aspartic acid racemate mixture

is a crystalline mixture of equal amounts of D-aspartic acid and L-aspartic acid, and the racemic aspartic acid is a compound formed by D-aspartic acid and L-aspartic acid alternately arranged in the crystal lattices.

The aspartic acid copper complex can be prepared according to the following scheme.

In a preparation scheme, 1 chemical molar equivalent of aspartic acid is added to a 15% (mass in percentage) aqueous solution containing 2 chemical molar equivalents of sodium hydroxide at room temperature. The reaction liquid is stirred to clarification, cooled to room temperature and then slowly added dropwise to a 40% (mass in percentage) aqueous solution containing 1 chemical molar equivalent of copper sulphate pentahydrate. After the dropwise addition, the resulting reaction liquid is further stirred to yield a blue solid. The reaction liquid is filtered, and the filter cake is washed with water and then dried by heating to obtain a solid product.

In one embodiment, the sodium hydroxide can be replaced with an equal molar equivalent of potassium hydroxide.

In one embodiment, the copper sulphate pentahydrate can be replaced with an equal molar equivalent of copper chloride and a hydrate thereof, copper bromide and a hydrate thereof, copper nitrate and a hydrate thereof, etc.

The complex involved in the present invention is an aspartic acid-copper (Cu : Asp = 1 : 1) hydrate. Using structural identification techniques, the inventors have determined that as an integral part of a crystal structure, m water molecules contained in the complex form a stable crystal structure with a copper ion and an Asp, and the remaining n water molecules are combined with the crystal structure according to a non-chemical equivalent.

Further, the reaction liquid is stirred at a low, medium or high stirring rate for 0 to 4 hours, and with regard to the solid product $[(Cu(II))(Asp)(H_2O)_m]\cdot n(H_2O)$, m is any integer from 0 to 10, and n is any value from 0 to 10.

In some embodiments, m is any integer from 0 to 2.

In some embodiments, the drying by heating is drying under reduced pressure, with the heating temperature of 60°C-110°C and the pressure of the drying oven of 0-0.1 MPa, and the solid product $[(Cu(II))(Asp)(H_2O)_m]\cdot (H_2O)_n$ is an aspartic acid copper complex where n is any value selected from 0 to 1.

In some embodiments, the drying by heating is drying under reduced pressure, with the heating temperature of 110°C-150°C and the pressure of the drying oven of 0-0.1 MPa, and the solid product $[(Cu(II))(Asp)(H_2O)_m]\cdot (H_2O)_n$ is an aspartic acid copper complex where m and n are independently 0.

In some embodiments, the 1 chemical molar equivalent of aspartic acid is L-aspartic acid, and the product is $[(Cu(II))(L\text{-}Asp)(H_2O)_m]\cdot (H_2O)_n$.

In some embodiments, the 1 chemical molar equivalent of aspartic acid is DL-aspartic acid, and the product is $[(Cu(II))(DL\text{-}Asp)(H_2O)_m]\cdot (H_2O)_n$.

In some embodiments, the aspartic acid copper complex has any one of the structures as shown below:

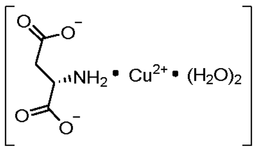, 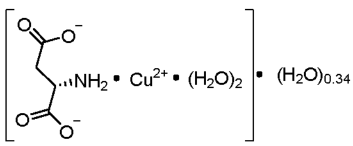, 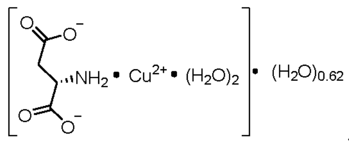,

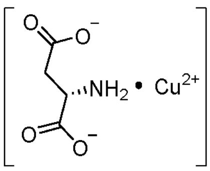, 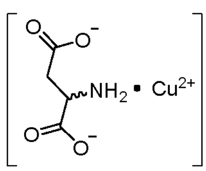, 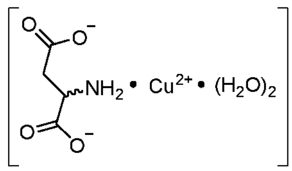,

and

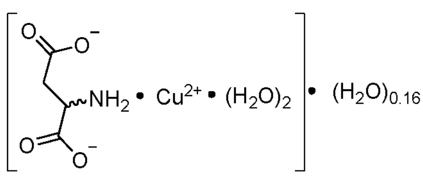.

The present invention provides a feed composition comprising at least one aspartic acid copper complex, and at least one of the auxiliary agents acceptable in feeds, pharmacy or foods.

The term "composition" involved in the present invention refers to a collection of compounds, which collection

comprises one or more compounds that are used as the active ingredients.

In the present invention, the term "comprise" is an open-ended expression that encompasses both the content explicitly indicated in the present invention and the content from other aspects.

In the present invention, the phrase "acceptable in feeds, pharmacy or foods" means that a substance or composition must be suitable in terms of chemistry or toxicology and related to the feed, drug, food or the farmed edible animals.

Optionally, the auxiliary agents comprise a carrier, a diluent, an excipient, and a vehicle that are commonly used in the industry of feeds, pharmacy or foods, or a combination thereof.

In the present invention, the term "carrier" refers to a substance that is acceptable in feeds, has the ability to carry the active ingredients and improve their dispersibility, and exhibits good chemical stability and adsorption. The carrier can be divided into organic carriers and inorganic carriers. The organic carrier generally refers to a material containing much crude fibre, and includes, but is not limited to, corn flour, corn cob powder, wheat bran, rice husk powder, defatted rice bran, rice mill by-product, corn stalk powder, peanut shell powder, etc. The inorganic carrier generally refers to minerals, which is primarily divided into calcium salts and oxides of silicon, and is used in the preparation of a trace element premix. The inorganic carrier includes, but is not limited to, calcium carbonate, silicates, vermiculite, zeolite, sepiolite, etc.

The term "diluent" involved in the present invention refers to a substance that enables additive raw materials to uniformly distribute in materials, dilutes high-concentration additive raw materials into a low-concentration premixed agent or premix, separates ingredients in trace amounts from each other and reduces the interaction between active ingredients, so as to increase the stability of the active ingredients without affecting the physico-chemical properties thereof. The diluent is divided into organic diluents and inorganic diluents. Common organic diluents include, but are not limited to, corn flour, degermed corn flour, dextrose (glucose), sucrose, semolina with bran, stir-fried soybean powder, wheat middling, corn gluten meal, etc. Commonly used inorganic diluents include, but are not limited to, limestone, calcium dihydrogen phosphate, shell powder, kaolin (white clay), salt and sodium sulphate.

The excipient is a wetting agent that induces the inherent viscosity of a substance, an adhesive that binds the substances together, a disintegrating agent that breaks the entire sheet of a substance into many fine particles, a retention aid that reduces the friction between particles, or an anti-sticking agent that prevents materials from adhesion. The excipient includes, but is not limited to, magnesium stearate, talc powder, vegetable oil, magnesium lauryl sulphate, starch, starch slurry, water, inorganic salt, dextrin, powdered sugar, etc.

The term "vehicle" involved in the present invention refers to a solvent required for dissolving or dispersing solids, including but not limited to water, ethanol, glycerol, etc.

In some embodiments, the feed composition further comprises an additional animal feed additive.

The additional animal feed additive is a nutritive feed additive, a general feed additive, or a medicinal feed additive.

The nutritive feed additive refers to a small or trace amount of substances that are added to formula feeds to balance feed nutrients, improve feed utilization, and directly exert nutritional effects on animals. The nutritive feed additive includes amino acids, amino acid salts and analogues thereof, vitamins and vitamin-like substances, mineral elements and complexes (chelates) thereof, microbial enzyme preparations or non-protein nitrogen.

The general feed additive, also referred to as a non-nutritive additive, refers to some non-nutritive substances that are added to feeds to improve feed utilization and ensure feed mass and quality, and are beneficial to animal health or metabolism. The general feed additive includes growth promoters, deworming agents, flavourings and attractants, feed conditioning agents, feed formulation agents, feed preservatives and Chinese herbal medicine additives.

Specifically, the medicinal feed additive includes, but is not limited to, a premixed veterinary drug that has the functions of preventing animal diseases and promoting animal growth and can be added to feeds and mixed with a carrier or diluent for long-term use.

In some embodiments, the feed composition may include feed raw materials, which are selected from feed substances derived from animals, plants, microorganisms or minerals but are not feed additives, and can be used to process and prepare feeds.

The animal feed raw materials are equivalent to raw materials acceptable in feeds. The animal feed raw materials are specifically feed substances, such as grains and processed products thereof; oilseeds and processed products thereof; leguminous crop seeds and processed products thereof; stem tubers, root tubers and processed products thereof; other seed and fruit products and processed products thereof; forage, roughage and processed products thereof; other plants, algae and processed products thereof; dairy products and by-products thereof; terrestrial animal products and by-products thereof; fish, other aquatic organisms and by-products thereof; minerals, microbial fermentation products and by-products thereof; and other feed raw materials.

In some embodiments, the feed composition is a premixed feed of additive, a concentrated feed, a formula feed, or a concentrate supplement.

The premixed feed of additive refers to a uniform mixture prepared from nutritive feed additives (main ingredients), which comprise any two or more of mineral trace elements, vitamins, microorganisms and amino acids, and the aspartic acid copper complex provided by the present invention or other feed additives, carriers and (or) diluents according to a given proportion, wherein the nutritive feed additives are present in a content that can meet the basic nutritional requirements of an applicable animal within its specific physiological stage, and the recommended dosage of aspartic acid copper complex in the formula feed, concentrate supplement or animal drinking water ranges from 5 mg/kg to 300 mg/kg calculated in terms of copper element.

The concentrated feed refers to a feed primarily prepared from proteins, minerals and feed additives according to a certain proportion.

The formula feed refers to a feed prepared from a variety of feed raw materials and feed additives according to a certain proportion depending on the nutritional requirements of the farmed animals.

The concentrate supplement refers to a feed prepared from a variety of feed raw materials and feed additives according to a certain proportion in order to supplement nutrition to herbivores.

The present invention also provides the use of the aspartic acid copper complex and the feed composition thereof in the preparation of an animal feed additive.

In some embodiments, the aspartic acid copper complex and the feed composition thereof are used in the preparation of an animal feed additive, wherein the animal feed additive is a livestock feed additive, a poultry feed additive, an aquaculture animal feed additive, or a pet feed additive.

The term "animal" involved in the present invention refers to human or farmed animals that cannot synthesize organic matters from inorganic matters, but can only utilize organic matters as food or feeds to perform vital activities such as ingestion, digestion, absorption, respiration, circulation, excretion, sensation, movement, and reproduction.

Optionally, the farmed animals include poultry, livestock, aquatic animals, and other animals that are raised in captivity and are legally captured including pets. Specifically, the poultry involved in the present invention are edible animals such as chickens, ducks, geese, pigeons, quails or turkeys at all stages of growth; the livestock involved in the present invention are edible animals such as pigs, cattle, goats, rabbits, and horses at all stages of growth; the aquaculture animals involved in the present invention are fish, shrimps, loaches, crabs or eels at all stages of growth; and the pets involved in the present invention include, but are not limited to, cats, dogs, rabbits, etc.

Specifically, the aspartic acid copper complex and the feed composition thereof are used in the preparation of a livestock feed additive, wherein the livestock include but are not limited to, pigs, cattle, goats, horses, rabbits, minks, etc. at all stages of growth.

Specifically, the aspartic acid copper complex and the feed composition thereof are used in the preparation of a poultry feed additive, wherein the poultry include, but are not limited to, chickens, ducks, geese, pigeons, etc. at all stages of growth.

In some embodiments, the animal feed additives prepared from the aspartic acid copper complex and the feed composition thereof are premixed agents, multi premixed agents, aqueous solutions, or granules.

The present invention also provides the use of the aspartic acid copper complex and the feed composition thereof in the preparation of an animal feed, wherein the animal feed is a livestock feed, a poultry feed, an aquaculture animal feed, or a pet feed.

Specifically, the aspartic acid copper complex and the feed composition thereof are used in the preparation of a livestock feed, wherein the livestock include, but are not limited to, pigs, cattle, goats, horses, rabbits, minks, etc. at all stages of growth.

Specifically, the aspartic acid copper complex and the feed composition thereof are used in the preparation of a poultry feed, wherein the poultry include, but are not limited to, chickens, ducks, geese, pigeons, etc. at all stages of growth.

In some embodiments, the prepared feed comprising the aspartic acid copper complex and the feed composition thereof is a single feed, a concentrated feed, a formula feed, a multi-premix, or a concentrate supplement.

Specifically, the formula feed is a complete formula feed.

In some embodiments, the recommended dosage of aspartic acid copper complex in a complete formula feed ranges from 5 mg/kg to 300 mg/kg calculated in terms of copper element.

Further, when the complete formula feed is for livestock, the recommended dosage of the aspartic acid copper complex ranges from 5 mg/kg to 250 mg/kg calculated in terms of copper element.

Specifically, the livestock are pigs, cattle, goats, horses, rabbits and minks, preferably pigs, at all stages of growth.

Still further, when the complete formula feed is for poultry, the recommended dosage of the aspartic acid copper complex ranges from 8 mg/kg to 200 mg/kg calculated in terms of copper element.

Specifically, the poultry are chickens, ducks, geese, pigeons, etc., preferably chickens and ducks, at all stages of growth.

The present invention further provides a method for improving the production performance of an animal, the method comprising: feeding the animal with a feed comprising the aspartic acid copper complex; alternatively, adding

the aspartic acid copper complex and the feed composition or feed additive thereof to the diet of the animal in an amount required for the growth of the corresponding animal and feeding the animal with the diet, wherein the recommended dosage of the aspartic acid copper complex, or the feed composition or additive thereof, ranges from 5 mg/kg to 300 mg/kg calculated in terms of copper element.

A skilled person with professional feeding knowledge (referred to as "breeders") know from experience that copper element deficiency in the diet of animals will retard the growth and development of animals, and it is necessary to timely supplement the copper supply to restore the normal growth and development of animals. In light of the animals' need for the copper element, breeders are free to choose different copper sources to feed animals. The copper sources include the aspartic acid copper complex provided by the present invention and the feed composition thereof, feed thereof or feed additive thereof. Breeders provide animals with animal food containing a sufficient amount of the aspartic acid copper complex according to the nutritional requirement for the copper element in animals at all stages of growth.

In some breeding schemes, the animal food includes, but is not limited to, an animal feed, a feed composition, a basal diet, etc.

In some specific breeding examples, the animal is livestock at all stages of growth, preferably pigs at all stages of growth. When provided with animal food comprising the aspartic acid copper complex and containing physiological necessary quantities of copper elements, the experimental pigs show improved food intake, average daily gain and feed conversion compared with the experimental pigs in the copper sulphate breeding example, aspartic acid breeding example or control breeding example without the drug.

In other specific breeding examples, the animal is poultry at all stages of growth, preferably chickens and ducks at all stages of growth. When provided with animal food comprising the aspartic acid copper complex and containing physiological necessary quantities of copper elements, the experimental chickens or ducks show improved feed conversion compared with the experimental chickens or ducks in the copper sulphate breeding example, aspartic acid breeding example or control breeding example, and when the experimental amount of the aspartic acid copper complex reaches 100 to 300 mg/kg, a poisoning phenomenon similar to that observed among the animals in the high-level copper sulphate breeding example is not observed among the experimental chickens.

It can be seen therefrom that in terms of animal growth performance improvement, the aspartic acid copper complex provided by the present invention can not only meet the growth requirement of animals, but also significantly improve the production performance of animals, compared with an inorganic copper source.

The embodiments of the present application will be described in detail with reference to examples below; however, it would be understood by those skilled in the art that the examples below are only used to illustrate the present application, and should not be considered as limiting the scope of the present application. If no specific conditions are indicated in the examples, conventional conditions or the conditions suggested by the manufacturer shall be followed. The reagents or instruments used therein for which manufacturers are not specified are all conventional products that are commercially available.

**Example A** Preparation of aspartic acid copper complex

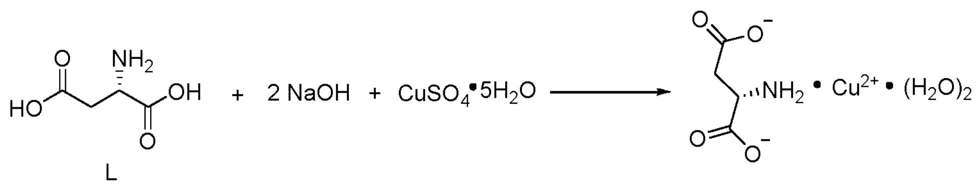

Formula II

Those skilled in the pertinent field will recognize that other methods for preparing the L-aspartic acid copper complex of the present application are considered as falling within the scope of the present application. For example, according to the present application, those unexemplified L-aspartic acid copper complexes can be successfully synthesized by those skilled in the pertinent field by means of modified methods, for example by means of using other reagents or making some conventional modifications to the reaction conditions.

At room temperature, 50 g of L-aspartic acid was added to a reaction flask containing 30.99 g of sodium hydroxide in 200 mL of water. The mixture was stirred to dissolution and clarification, and cooled to room temperature. The prepared aqueous sodium L-aspartate solution was slowly added dropwise to a reaction flask containing 93.8 g of copper sulphate pentahydrate and 250 mL of water (a clear aqueous solution of copper sulphate pentahydrate). A blue solid was yielded. The aqueous sodium L-aspartate solution was added dropwise over about 1.0 h, and then the reaction

liquid was stirred for another 4.0 h and filtered. The filter cake was washed with 100 mL of water and dried at 105°C for 16 h to afford the product (84.6 g, yield: 94.9%) as a blue solid.

Element analysis of product: Cu 26.40%, C 20.89%, H 4.04%, and N 6.07%.

Analysis of infrared diffraction spectrum detection results: FIG. 1 shows the infrared diffraction spectrum detection results of the product. FIG. 2 shows the infrared diffraction spectrum detection results of the mixture obtained by simply mixing the raw materials aspartic acid and copper sulphate pentahydrate, which are required for the preparation of the product in the above-mentioned preparation scheme, according to the feed ratio. It can be seen from the comparison between FIG. 1 and FIG. 2 that there are a significantly reduced number of characteristic absorption peaks in FIG. 1, indicating that the symmetry is higher than that of aspartic acid, and the amino acid characteristic absorption peak at 2083 cm-1 in FIG. 2 is not present in the absorption peaks in FIG. 1; there is a strong and broad absorption peak at 2500 cm-1 to 3400 cm-1 in FIG. 2, whereas there is a relatively narrow absorption peak at 3100 cm-1 to 3400 cm-1 in FIG. 1, indicating that free - OH is not present in the product; and there are coordination characteristic peaks of carboxyl groups at around 1600 cm-1 and 1400 cm-1 in FIG. 1, indicating the coordination between the metal and the carboxyl group.

It is verified by the structural identification technique, element analysis method and infrared diffraction spectrometry that the product obtained using the preparation scheme as shown in this example is an L-aspartic acid copper complex having a structural formula of

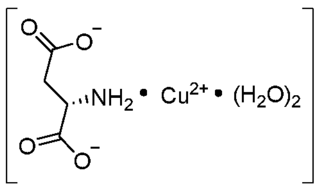

($[(Cu(II))(L\text{-}Asp)(H_2O)_2]$, compound 1).

Additionally, in some batches, the product from the above-mentioned preparation scheme is dried under reduced pressure at 60°C-140°C. It is verified by the structural identification technique, element analysis method, infrared diffraction spectrometry and thermogravimetric analysis technique that the products obtained further include those having structural formulas of

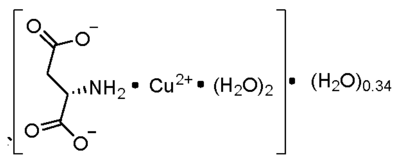

($[(Cu(II))(L\text{-}Asp)(H_2O)_2]\cdot (H_2O)_{0.34}$, compound 2, blue solid, containing 2.74% non-stoichiometric water) and of

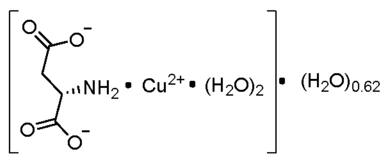

($[(Cu(II))(L\text{-}Asp)(H_2O)_2]\cdot (H_2O)_{0.62}$, compound 3, blue solid, containing 4.62% non-stoichiometric water). Furthermore, when the product is dried under reduced pressure at 150°C-209°C, it is verified by the structural identification technique, element analysis method, infrared diffraction spectrometry and thermogravimetric analysis technique that the product obtained has a structural formula of

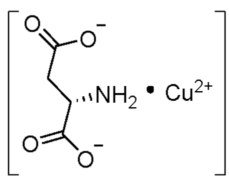

($[(Cu(II))(L\text{-}Asp)]$, compound 4, off-white solid).

Moreover, the inventors have found that using an aspartic acid racemate mixture or a racemic aspartic acid in place of L-aspartic acid in the above-mentioned preparation scheme, the following products can be respectively obtained following the preparation processes of compound 1, compound 4 and compound 2:

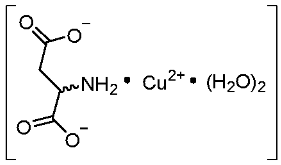

([(Cu(II))(DL-Asp)($H_2O$)$_2$], compound 5, blue solid),

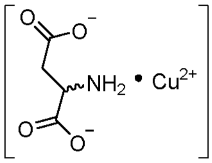

([(Cu(II))(DL-Asp)], compound 6, off-white solid), and

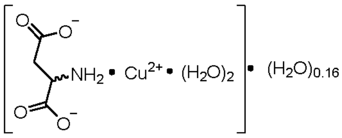

([(Cu(II))(DL-Asp)($H_2O$)$_2$]· ($H_2O$)$_{0.16}$, compound 7, blue solid, containing 1.23% non-stoichiometric water).

Example B Breeding experiment

B-1 Trial for growth-promoting effect of aspartic acid copper complex

(1) Experimental materials

Experimental animals: 900 one-day-old Lingnan yellow-feathered, fast-growing, and large-sized broilers; 150 weaned piglets;

Feeds: basal feed for chickens and basal feed for pigs that contain no antimicrobial drugs, copper sources and growth promoters;

Experimental samples: compound 1, compound 2, compound 3, compound 4, compound 5, compound 6, compound 7,

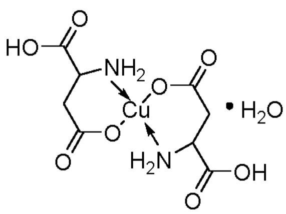

(compound 8, prepared according to the method provided in Chemical World, 2005, 02, 94-96), L-aspartic acid, and copper sulphate pentahydrate.

(2) Experimental method

a. Trial for growth-promoting effect of aspartic acid copper complex in Lingnan yellow-feathered, fast-growing, and large-sized broilers

900 one-day-old Lingnan yellow-feathered, fast-growing, and large-sized broilers were randomly divided into 18 groups (50 broilers/group). In the control group, only a physiological recommendation amount (5 mg/kg) of copper sulphate was added to the feeds; in other groups, different experimental samples as shown in Table 1 were added to the feeds without adding any other copper element supplement. The broilers had free access to food. The weight gain and feed conversion of the experimental chickens (aged from 1 to 21 days) in each experimental group were recorded. Copper sulphate and aspartic acid copper complex were compared in terms of their effects on promoting the growth of broilers.

Table 1 Groups of the trial for growth-promoting effect of aspartic acid copper complex in broilers

| Group | Number of animal | Average initial weight (g) | Growth promoter | Concentration (mg/kg)* |
|---|---|---|---|---|
| Control group | 50 | 50 | Copper sulphate pentahydrate | 5 |
| Copper sulphate pentahydrate-150 | 50 | 50 | Copper sulphate pentahydrate | 150 |
| Copper sulphate pentahydrate-100 | 50 | 50 | Copper sulphate pentahydrate | 100 |
| Copper sulphate pentahydrate-50 | 50 | 50 | Copper sulphate pentahydrate | 50 |
| Compound 1-300 | 50 | 50 | Compound 1 | 300 |
| Compound 1-200 | 50 | 50 | Compound 1 | 200 |
| Compound 1-100 | 50 | 50 | Compound 1 | 100 |
| Compound 1-50 | 50 | 50 | Compound 1 | 50 |
| Compound 1-35 | 50 | 50 | Compound 1 | 35 |
| Compound 1-5 | 50 | 50 | Compound 1 | 5 |
| Compound 2-50 | 50 | 50 | Compound 2 | 50 |
| Compound 3-50 | 50 | 50 | Compound 3 | 50 |
| Compound 4-50 | 50 | 50 | Compound 4 | 50 |
| Compound 5-50 | 50 | 50 | Compound 5 | 50 |
| Compound 6-50 | 50 | 50 | Compound 6 | 50 |
| Compound 7-50 | 50 | 50 | Compound 7 | 50 |
| Compound 8-50 | 50 | 50 | Compound 8 | 50 |
| L-aspartic acid | 50 | 50 | L-aspartic acid | 105 |
| *: different growth promoters were added at amounts calculated in term of copper ion of the compounds, and the amount added in the L-aspartic acid group was consistent with the molar mass of L-aspartic acid contained in the case of the compound 1-50 group. | | | | |

b. Trial for growth-promoting effect of aspartic acid copper complex in pigs

150 weaned piglets were grouped according to Table 2 (10 piglets/group). In the control group, only a physiological recommendation amount (8 mg/kg) of copper sulphate was added to the feeds; in other groups, different experimental samples as shown in Table 2 were added to the feeds without adding any other copper element supplement. The piglets had free access to food. The weight gain and feed conversion of the experimental pigs in each experimental

group were recorded within 30 days after weaning. Copper sulphate and aspartic acid copper complex were compared in terms of their effects on promoting the growth of pigs.

Table 2 Groups of the trial for growth-promoting effect of aspartic acid copper complex in pigs

| Group | Number of animal | Average initial weight (kg) | Growth promoter | Concentration (mg/kg)* |
|---|---|---|---|---|
| Control group without growth promoter | 10 | 8.36 | Copper sulphate pentahydrate | 8 |
| Copper sulphate pentahydrate-250 | 10 | 8.31 | Copper sulphate pentahydrate | 250 |
| Compound 1-150 | 10 | 8.42 | Compound 1 | 150 |
| Compound 1-100 | 10 | 8.38 | Compound 1 | 100 |
| Compound 1-50 | 10 | 8.25 | Compound 1 | 50 |
| Compound 1-35 | 10 | 8.31 | Compound 1 | 35 |
| Compound 1-8 | 10 | 8.44 | Compound 1 | 8 |
| Compound 2-50 | 10 | 8.35 | Compound 2 | 50 |
| Compound 3-50 | 10 | 8.32 | Compound 3 | 50 |
| Compound 4-50 | 10 | 8.40 | Compound 4 | 50 |
| Compound 5-50 | 10 | 8.51 | Compound 5 | 50 |
| Compound 6-50 | 10 | 8.37 | Compound 6 | 50 |
| Compound 7-50 | 10 | 8.44 | Compound 7 | 50 |
| Compound 8-50 | 10 | 8.29 | Compound 8 | 50 |
| L-aspartic acid | 10 | 8.43 | L-aspartic acid | 104 |
| *: different growth promoters were added at amounts calculated as the copper ion in the compounds; and the amount added in the L-aspartic acid group was consistent with the molar mass of L-aspartic acid contained in the case of the compound 1-50 group. | | | | |

(3) Experimental results

a. Trial for growth-promoting effect of aspartic acid copper complex in Lingnan yellow-feathered, fast-growing, and large-sized broilers

During the feeding experiment of Lingnan yellow-feathered, fast-growing, and large-sized broilers, in the second week of the experiment, the Lingnan yellow-feathered, fast-growing, and large-sized broilers in copper sulphate groups (150 mg/kg copper ions and 100 mg/kg copper ions, respectively) showed poisoning symptoms such as mussy feathers and dry skin, and by the end of the trial, a small number of chickens died. It indicated that the long-term use of a feed with copper sulphate at an addition amount of 100 mg/kg had toxic side effects on the broilers. In the copper sulphate group using 50 mg/kg copper ions, no clinically visible poisoning symptoms were observed; however, compared with the control group without drugs, during the trial, the group showed a similar relative weight gain rate which was 98.9%, but exhibited a 0.048 higher feed-to-meat ratio, suggesting that the feed conversion was not improved. In the experimental groups of compound 1, compound 2, compound 3, compound 4, compound 5, compound 6 and compound 7, the compounds substantially demonstrated good growth-promoting effects and the experimental chickens exhibited the normal physical characteristics. The relative weight gain rate was increased by 4.9%-19.6%, and the feed conversion was decreased by 2.95%-6.34%. Among them, the experimental group using compound 1 at an addition amount of 50-100 mg/kg exhibited a dose effect in the experimental results. With regard to the compound 4 group, the relative weight gain rate was increased by 7.2%, and the feed conversion was decreased by 3.23%. With regard to the L-aspartic acid group, the relative weight gain rate was increased by 4.2%, and the feed conversion was decreased by 0.32%. The experimental results suggest that the aspartic acid copper complexes have good safety and growth-promoting effects; however, aspartic acid copper complexes with different structures show different effects on the growth performance of the animals while provided at the same addition amounts (see Table 3 for details).

Table 3 Results of the trial for growth-promoting effect of aspartic acid copper complex in broilers

| Group | Number of animal (broiler/ broilers) | Survival rate (%) | Average weight gain (g) | Relativeweight gain rate (%) | Average feed consumption (g) | Feed-to-meat ratio |
|---|---|---|---|---|---|---|
| Control group without drug | 50 | 100 | 474 | - | 891 | 1.879 |
| Copper sulphate-150 | 50 | 88 | 427 | 90.1 | 818 | 1.915 |
| Copper sulphate-100 | 50 | 94 | 459 | 96.8 | 881 | 1.920 |
| Copper sulphate-50 | 50 | 100 | 469 | 98.9 | 923 | 1.969 |
| Compound 1-300 | 50 | 100 | 526 | 111.0 | 926 | 1.760 |
| Compound 1-200 | 50 | 100 | 567 | 119.6 | 1004 | 1.771 |
| Compound 1-100 | 50 | 100 | 546 | 115.2 | 970 | 1.777 |
| Compound 1-50 | 50 | 100 | 518 | 109.3 | 923 | 1.782 |
| Compound 1-30 | 50 | 100 | 511 | 107.8 | 919 | 1.798 |
| Compound 1-5 | 50 | 100 | 497 | 104.9 | 916 | 1.843 |
| Compound 2-50 | 50 | 100 | 509 | 107.4 | 908 | 1.783 |
| Compound 3-50 | 50 | 100 | 514 | 108.4 | 919 | 1.788 |
| Compound 4-50 | 50 | 100 | 508 | 107.2 | 924 | 1.819 |
| Compound 5-50 | 50 | 100 | 503 | 106.1 | 917 | 1.824 |
| Compound 6-50 | 50 | 100 | 510 | 107.6 | 912 | 1.789 |
| Compound 7-50 | 50 | 100 | 515 | 108.6 | 921 | 1.789 |
| Compound 8-50 | 50 | 100 | 478 | 100.8 | 895 | 1.872 |
| L-aspartic acid | 50 | 100 | 480 | 101.3 | 899 | 1.873 |

b. Trial for growth-promoting effect of aspartic acid copper complex in pigs

During the feeding trial of pigs, in the high-level copper sulphate pentahydrate group (250 mg/kg), an obvious growth-promoting effect was observed. During the experiment, the average weight gain was increased by 12.66% and the feed conversion was decreased by 7.8% compared with the control group without drugs. Compound 1, compound 2, compound 3, compound 4, compound 5, compound 6, compound 7 and compound 8 were used in this experiment. The first seven compounds all had growth-promoting effects on the experimental pigs. However, compound 1, compound 2, and compound 3, when used at the same doses, achieved about 2-fold improvement in the feed conversion of the experimental pigs compared with compound 4, and compound 1, compound 2, and compound 3 achieved similar feed conversion at lower doses than compound 4. In addition, when the experimental pigs were provided with compound 8 at this dose, their production performance was substantially close to that achieved in the control group. Additionally, compound 1 exhibited a dose effect on improving the production performance of the experimental pigs. When the compound was provided at a dose of 50 mg/kg, its growth-promoting effect and feed conversion rate were close to those achieved in the high-level copper sulphate group (Table 4). The results suggest that 50 mg/kg compound 1 can be used as a substitute for high-level copper sulphate in pig breeding.

Table 4 Results of the trial for growth-promoting effect of aspartic acid copper complex in pigs

| Group | Number of animal (pig/pigs) | Survival rate (%) | Average weight gain (kg) | Relative weight gain rate (%) | Total weight gain (kg) | Total feed consumption (kg) | Feed-to-meat ratio |
|---|---|---|---|---|---|---|---|
| Control group without growth promoter | 10 | 100 | 10.90 | 100 | 109 | 250.28 | 2.296 |
| Copper sulphate pentahydrate-250 | 10 | 100 | 12.28 | 112.66 | 122.8 | 260.09 | 2.118 |
| Compound 1-200 | 10 | 100 | 12.34 | 113.21 | 123.4 | 258.28 | 2.093 |
| Compound 1-100 | 10 | 100 | 12.21 | 112.02 | 122.1 | 257.86 | 2.112 |
| Compound 1-50 | 10 | 100 | 12.12 | 111.19 | 121.2 | 257.08 | 2.121 |
| Compound 1-30 | 10 | 100 | 11.66 | 106.97 | 116.6 | 257.09 | 2.205 |
| Compound 1-8 | 10 | 100 | 11.26 | 103.30 | 112.6 | 255.34 | 2.268 |
| Compound 2-50 | 10 | 100 | 12.30 | 112.8 | 123.0 | 260.21 | 2.116 |
| Compound 3-50 | 10 | 100 | 11.92 | 109.4 | 119.2 | 251.97 | 2.114 |
| Compound 4-50 | 10 | 100 | 11.67 | 107.06 | 116.7 | 257.63 | 2.208 |
| Compound 5-50 | 10 | 100 | 11.83 | 108.5 | 118.3 | 258.35 | 2.184 |
| Compound 6-50 | 10 | 100 | 12.08 | 110.8 | 120.8 | 262.46 | 2.173 |
| Compound 7-50 | 10 | 100 | 12.16 | 111.6 | 121.6 | 267.33 | 2.198 |
| Compound 8-50 | 10 | 100 | 11.06 | 101.5 | 110.6 | 253.37 | 2.291 |
| L-aspartic acid | 10 | 100 | 11.04 | 101.28 | 110.4 | 252.27 | 2.285 |

**B-2** Use of aspartic acid copper complex in aquaculture feeds

(1) Experimental animals and materials

Experimental fishes: The experimental fishes used were black carp (subyearlings), which were provided by the Dafeng fish nursery (Huizhou city, Guangdong province). Healthy and active fingerling black carps (uniform in size) were fed for 4 weeks in a big net cage ($4 \times 2 \times 1.5$ $m^3$) and then the formal breeding experiment was carried out. The experimental systems were small floating net cages ($1.1 \times 1.1 \times 1.1$ $m^3$ in size). Each small net cage was provided with an air-filling head, which allowed for continuous inflation of the cages for 24 h a day. The small net cages and the acclimatization net cage were all placed in a 3500 $m^2$ pond in the experimental field. The pond had a water depth of about 1.5 meters, and the water in the pond was fully aerated underwater. During the trial, 480 black carps (fasted for 1 d) were randomly divided into 10 groups, with 4 replicates for each group and 12 fishes in each replicate. After weighing as a whole, the black carps were randomly placed into 28 net cages and fed with different experimental feeds.

Experimental feeds: The experimental feeds were formulated following the formula in Table 5. According to Table 6, different copper element supplements (calculated in terms of the copper ions) were added to the feeds in different experimental groups, respectively. The feed raw materials were subjected to ultrafine grinding and then granulated into floating expanded feeds (3 mm) using an expansion machine from Jiangsu Muyang Corporation. The demoulding temperature was 130°C. 3% soybean oil was externally sprayed using an oil spraying device. The finished product was sealed and stored in a cool place for later use.

Table 5: Black carp feed formula for trial and chemical ingredients (%wt.)

| Raw material composition | Content (%) | Raw material composition | Content (%) |
|---|---|---|---|
| Fish meal | 9.0 | Soybean oil | 3.0 |
| Casing meal | 3.0 | Phospholipid-rapeseed meal | 9.0 |

(continued)

| Raw material composition | Content (%) | Raw material composition | Content (%) |
|---|---|---|---|
| Soybean meal | 12.0 | Gluten powder | 4.0 |
| Rapeseed meal | 12.0 | Blood cell powder | 2.0 |
| Monosodium glutamate protein | 3.0 | Vc-phosphate | 0.1 |
| Wheat middling | 12.6 | Calcium dihydrogen phosphate | 1.8 |
| Flour | 17.0 | Choline chloride | 0.2 |
| Bentonite | 0.70 | Various vitamins | 0.1 |
| Rice bran | 10.0 | Premixed agent of trace minerals | 0.5 |

(2) Experimental method

Experimental management: During the trial, the carps were manually fed in a restricted dose. The feeding dose was adjusted once a week, and the doses for all groups (based on the initial body weight) were completely consistent. The carps were fed twice a day (at 7:30 and 15:00). The experiment lasted for 8 weeks. During the trial, the water quality was regularly monitored, and throughout the breeding, the water temperature was kept at 26.88 ± 3.08°C, DO > 5.0 mg O $L^{-1}$, pH 7.8, ammonia nitrogen < 0.50 mg N $L^{-1}$, and nitrite-nitrogen < 0.05 mg N $L^{-1}$.

Parametric statistics: During the trial, the carps in each net cage were weighed as a whole one day after discontinuation of feeding, and the average weight gain (g) and feed-to-meat ratio were calculated. The calculation formulas are as follows:

$$\text{Average weight gain (g)} = \text{Average final weight} - \text{Average initial weight;}$$

$$\text{Feed-to-meat ratio} = \text{Food intake/ Weight gain of fishes;}$$

(3) Experimental results

The results of the trial for growth-promoting effect of the aspartic acid copper complex in fishes can be seen in Table 6. The results show that the aspartic acid copper complex provided by the present invention can significantly improve the daily weight gain and feed-to-meat ratio of the experiment fishes within the range of the physiological requirement amount limits for the animal. Compared with copper sulphate at an equivalent dose, the aspartic acid copper complex provided by the present invention has superior effects in improving the production performance of cultured fishes.

Table 6 Application effects of aspartic acid copper complex in aquaculture feeds

| Group | Number of animal | Additives | Dose (mg/kg) | Average weight gain (g) | Feed-to-meat ratio |
|---|---|---|---|---|---|
| 1 | 48 | Control group | - | 325 | 1.538 |
| 2 | 48 | Copper sulphate | 30 | 343 | 1.458 |
| 3 | 48 | Compound 1 | 30 | 357 | 1.401 |
| 4 | 48 | Compound 2 | 30 | 368 | 1.359 |
| 5 | 48 | Compound 3 | 30 | 361 | 1.385 |
| 6 | 48 | Compound 4 | 30 | 344 | 1.453 |
| 7 | 48 | Compound 5 | 30 | 349 | 1.433 |
| 8 | 48 | Compound 6 | 30 | 353 | 1.416 |
| 9 | 48 | Compound 7 | 30 | 350 | 1.429 |
| 10 | 48 | Compound 8 | 30 | 338 | 1.479 |
| Notes: the concentration of the copper-containing preparation among the additives was calculated in terms of the copper ions; and the control group was an experimental group without adding any copper-containing preparation. | | | | | |

## Claims

1. An aspartic acid copper complex having a chemical structure of $[(Cu(II))(Asp)(H_2O)_m]\cdot(H_2O)_n$, wherein Asp is L-Asp or DL-Asp, m is any integer from 0 to 10, and n is any value from 0 to 10.

2. The aspartic acid copper complex according to claim 1, **characterised in that** m is 2, and n is any value from 0 to 0.62.

3. The aspartic acid copper complex according to claim 1, **characterised in that** the aspartic acid copper complex has any one of the structures as shown below:

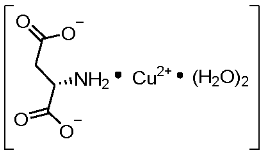,
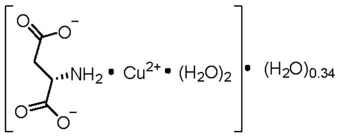,
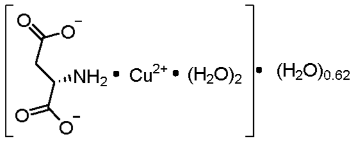,

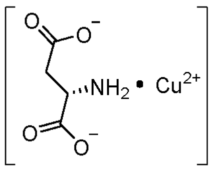,
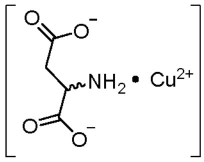,
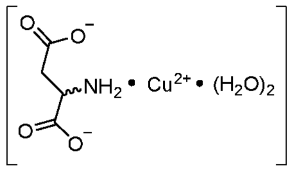,

and

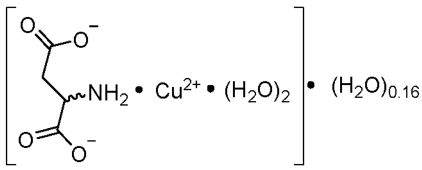.

4. Use of the aspartic acid copper complex according to any one of claims 1-3 in the preparation of an animal feed additive.

5. The use according to claim 4, **characterised in that** the animal feed additive is a feed additive suitable for livestock, poultry, aquatic animals, or pets at all stages of growth.

6. The use according to claim 4, **characterised in that** the recommended dosage of the aspartic acid copper complex, calculated in terms of copper element, ranges from 5 mg/kg to 300 mg/kg.

7. The use according to claim 6, **characterised in that** the aspartic acid copper complex is a copper element supplement and the recommended dosage thereof in an animal feed, calculated in terms of copper element, ranges from 5 mg/kg to 35 mg/kg.

8. The use according to claim 5, **characterised in that** the aspartic acid copper complex is a growth promoter for an animal.

9. The use according to claim 8, **characterised in that** the recommended dosage of the aspartic acid copper complex in an animal feed, calculated in terms of copper element, ranges from 5 mg/kg to 250 mg/kg, and the animal is a pig, a chicken or a duck at all stages of growth.

10. A feed composition, **characterised in that** the feed composition comprises at least one aspartic acid copper complex according to any one of claims 1-3, and at least one of a carrier, an excipient, a diluent and a vehicle acceptable in pharmacy, foods or feeds.

11. The feed composition according to claim 10, **characterised in that** the feed composition further comprises an

additional animal feed additive.

**12.** The feed composition according to claim 10 or 11, **characterised in that** the feed composition further comprises a feed raw material.

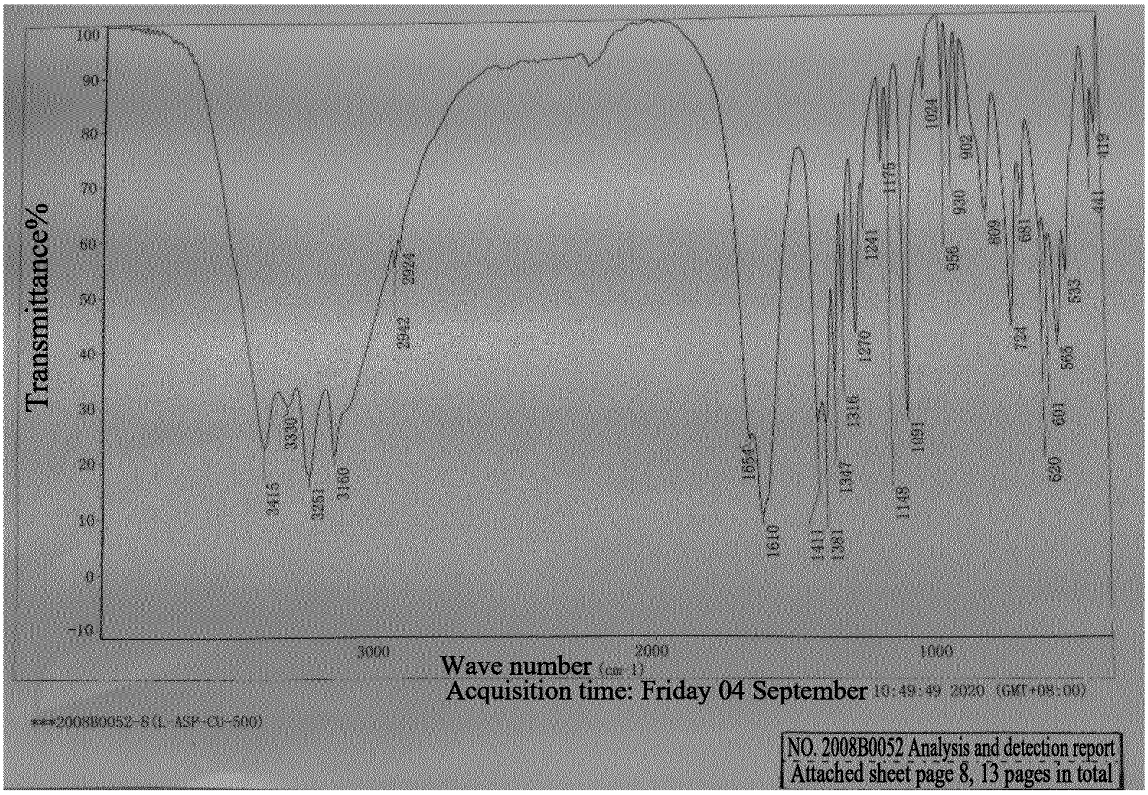
Transmittance%
Wave number (cm-1)
Acquisition time: Friday 04 September 10:49:49 2020 (GMT+08:00)
***2008B0052-8(L-ASP-CU-500)
NO. 2008B0052 Analysis and detection report
Attached sheet page 8, 13 pages in total

FIG. 1

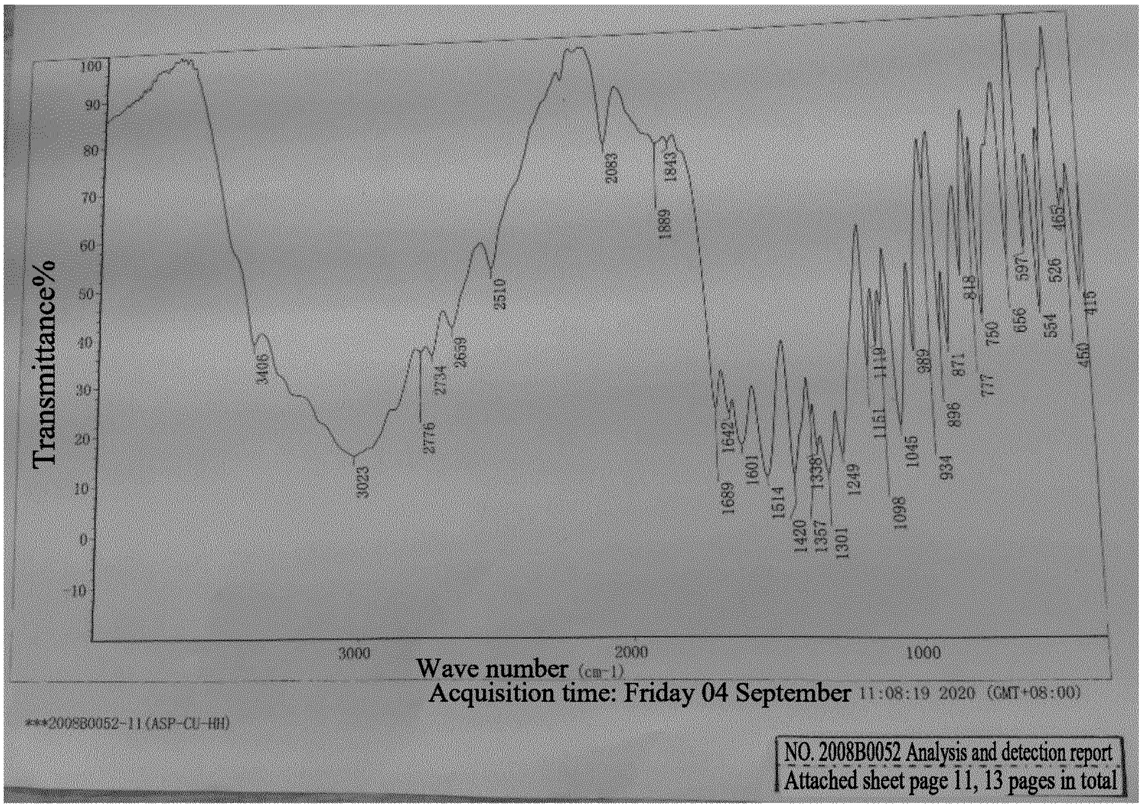
Transmittance%
Wave number (cm-1)
Acquisition time: Friday 04 September 11:08:19 2020 (GMT+08:00)
***2008B0052-11(ASP-CU-HH)
NO. 2008B0052 Analysis and detection report
Attached sheet page 11, 13 pages in total

FIG. 2

**INTERNATIONAL SEARCH REPORT**

International application No.
**PCT/CN2022/078264**

**A. CLASSIFICATION OF SUBJECT MATTER**

A23K 20/20(2016.01)i

According to International Patent Classification (IPC) or to both national classification and IPC

**B. FIELDS SEARCHED**

Minimum documentation searched (classification system followed by classification symbols)

A23K

Documentation searched other than minimum documentation to the extent that such documents are included in the fields searched

Electronic data base consulted during the international search (name of data base and, where practicable, search terms used)

CNTXT; CJFD; CNKI; DWPI; ENTXTC; WPABSC: 天冬氨酸, 铜, aspartic acid, copper, aspartate

**C. DOCUMENTS CONSIDERED TO BE RELEVANT**

| Category* | Citation of document, with indication, where appropriate, of the relevant passages | Relevant to claim No. |
|---|---|---|
| PX | CN 113170837 A (PENG, Xianfeng) 27 July 2021 (2021-07-27)<br>claims 1-12 | 1-12 |
| X | US 2004077714 A1 (ZINPRO CORP) 22 April 2004 (2004-04-22)<br>claims 1-14, description paragraphs 23, 54-57 | 1-12 |
| X | WO 2010074602 A1 (VORONIN SERGEI PETROVICH et al.) 01 July 2010 (2010-07-01)<br>claim 1 | 1-12 |
| A | EA 201900072 A2 (JOINT STOCK COMPANY BIOAMID) 31 August 2020 (2020-08-31)<br>entire document | 1-12 |
| A | WO 2020159399 A1 (AKTSIONERNOE OBSCHESTVO "BIOAMID") 06 August 2020 (2020-08-06)<br>entire document | 1-12 |
| A | CN 1484971 A (SHIJIAZHUANG CITY KEXING ANIMAL HEALTHCARE PRODUCT LTD.) 31 March 2004 (2004-03-31)<br>entire document | 1-12 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **19 April 2022** | **09 May 2022** |
| Name and mailing address of the ISA/CN<br>**China National Intellectual Property Administration (ISA/CN)**<br>**No. 6, Xitucheng Road, Jimenqiao, Haidian District, Beijing 100088, China** | Authorized officer |
| Facsimile No. **(86-10)62019451** | Telephone No. |

Form PCT/ISA/210 (second sheet) (January 2015)

**INTERNATIONAL SEARCH REPORT**
**Information on patent family members**

International application No.
**PCT/CN2022/078264**

| Patent document cited in search report | Publication date (day/month/year) | Patent family member(s) | Publication date (day/month/year) |
|---|---|---|---|
| CN 113170837 A | 27 July 2021 | None | |
| US 2004077714 A1 | 22 April 2004 | TW 200410639 A | 01 July 2004 |
| | | PL 373067 A1 | 08 August 2005 |
| | | IL 164894 D0 | 18 December 2005 |
| | | TR 200403625 T2 | 21 April 2005 |
| | | IN 3383DELNP2004 A | 20 March 2009 |
| | | MX PA05003809 A | 08 June 2005 |
| | | KR 20050037437 A | 21 April 2005 |
| | | BR 0311654 A | 15 March 2005 |
| | | DE 60325878 D1 | 05 March 2009 |
| | | US 2004096482 A1 | 20 May 2004 |
| | | ES 2318199 T3 | 01 May 2009 |
| | | EP 1556336 A1 | 27 July 2005 |
| | | AT 420849 T | 15 January 2009 |
| | | CN 1668577 A | 14 September 2005 |
| | | NZ 536677 A | 27 October 2006 |
| | | PT 1556336 E | 08 April 2009 |
| | | JP 2006503092 A | 26 January 2006 |
| | | DK 1556336 T3 | 14 April 2009 |
| | | AU 2003262869 A1 | 04 May 2004 |
| | | CA 2488696 A1 | 29 April 2004 |
| | | WO 2004035524 A1 | 29 April 2004 |
| | | US 2004097748 A1 | 20 May 2004 |
| | | ZA 200408841 B | 25 January 2006 |
| WO 2010074602 A1 | 01 July 2010 | UA 103913 C2 | 10 December 2013 |
| | | EA 201100628 A1 | 30 December 2011 |
| | | RU 2008151504 A | 27 June 2010 |
| EA 201900072 A2 | 31 August 2020 | None | |
| WO 2020159399 A1 | 06 August 2020 | EP 3919473 A1 | 08 December 2021 |
| CN 1484971 A | 31 March 2004 | None | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- *Chemical World,* 2005, vol. 02, 94-96